Europäisches Patentamt

European Patent Office

Office européen des brevets

(19)

(11) EP 0 640 139 B1

(12) EUROPÄISCHE PATENTSCHRIFT

(45) Veröffentlichungstag und Bekanntmachung des Hinweises auf die Patenterteilung:
16.07.1997 Patentblatt 1997/29

(21) Anmeldenummer: 93911444.3

(22) Anmeldetag: 03.04.1993

(51) Int Cl.6: **C12N 15/76**
// C12N15/57, C12N15/55, C12N15/56

(86) Internationale Anmeldenummer:
PCT/EP93/00833

(87) Internationale Veröffentlichungsnummer:
WO 93/20215 (14.10.1993 Gazette 1993/25)

(54) **VERFAHREN ZUR HERSTELLUNG REKOMBINANTER PROTEINE IN STREPTOMYCETEN**

PROCESS FOR PRODUCING RECOMBINANT PROTEINS IN STEPTOMYCETES

PROCEDE DE FABRICATION DE PROTEINES RECOMBINANTES DANS DES STREPTOMYCITES

(84) Benannte Vertragsstaaten:
AT BE CH DE DK ES FR GB GR IE IT LI LU NL PT SE

(30) Priorität: 07.04.1992 DE 4211517

(43) Veröffentlichungstag der Anmeldung:
01.03.1995 Patentblatt 1995/09

(73) Patentinhaber: BOEHRINGER MANNHEIM GMBH
68305 Mannheim (DE)

(72) Erfinder:
• PIEPERSBERG, Wolfgang
D-42349 Wuppertal (DE)
• WEISS, Angela
D-97999 Igersheim (DE)
• RÖSSLER, Carola
D-63110 Rodgau (DE)

(74) Vertreter: Fouquet, Herbert, Dr. et al
c/o Boehringer Mannheim GmbH,
Patentabteilung,
Sandhoferstrasse 116
68298 Mannheim (DE)

(56) Entgegenhaltungen:
WO-A-88/07079

• GENE Bd. 41, 1986, AMSTERDAM NL Seiten E357 - E368 BIBB, M.J. ET AL. 'Cloning and analysis of the promoter region of the erythromycin-resistance gene (ermE) of Streptomyces erythraeus' in der Anmeldung erwähnt
• PROCEEDINGS OF THE NATIONAL ACADEMY OF SCIENCES OF USA Bd. 84, Nr. 8, April 1987, WASHINGTON US Seiten 2130 - 2134 FORNWALD, J. ET AL. 'Two promoters, one inducible and one constitutive, control transcription of the Streptomyces lividans galactose operon in der Anmeldung erwähnt
• lividans galactose operon' in der Anmeldung erw hnt

SEQUENZ PROTOKOLL

(1) ALLGEMEINE INFORMATION:

  (i) ANMELDER:

    (A) NAME: Boehringer Mannheim GmbH
    (B) STRASSE: Sandhofer Str. 116
    (C) ORT: Mannheim
    (D) BUNDESLAND: Deutschland
    (F) POSTLEITZAHL: D-6800

  (ii) ANMELDETITEL: Verfahren zur Herstellung rekombinanter Proteine in Streptomyceten

  (iii) ANZAHL DER SEQUENZEN: 11

  (iv) COMPUTER-LESBARE FORM:

    (A) DATENTRÄGER: Floppy disk
    (B) COMPUTER: IBM PC compatible
    (C) BETRIEBSSYSTEM: PC-DOS/MS-DOS
    (D) SOFTWARE: PatentIn Release #1.0, Version #1.25 (EPA)

  (v) GEGENWÄRTIGE ANMELDEDATEN:
      ANMELDENUMMER: DE P 42 11 517.5

(2) INFORMATION ZU SEQ ID NO: 1:

  (i) SEQUENZ CHARAKTERISTIKA:

    (A) LÄNGE: 335 Basenpaare
    (B) ART: Nukleinsäure
    (C) STRANGFORM: Einzel
    (D) TOPOLOGIE: linear

  (ii) ART DES MOLEKÜLS: DNS (genomisch)

  (xi) SEQUENZBESCHREIBUNG: SEQ ID NO: 1:

```
AGATCTGGGG AATTCGAGCT CGGTACCAGC CCGACCCGAG CACGCGCCGG CACGCCTGGT   60

CGATGTCGGA CCGGAGTTCG AGGTACGCGG CTTGCAGGTC CAGGAAGGGG ACGTCCATGC  120

GAGTGTCCGT TCGAGTGGCG GCTTGCGCCC GATGCTAGTC GCGGTTGATC GGCGATCGCA  180

GGTGCACGCG GTCGATCTTG ACGGCTGGCG AGAGGTGCGG GGAGGATCTG ACCGACGCGG  240

TCCACACGTG GCACCGCGAT GCTGTTGTGG GCACAATCGT GCCGGTTGGT AGGATCCTCT  300

AGAGTCGACC GGCATGCAAG CTTGGCTGCA GATCT                             335
```

(2) INFORMATION ZU SEQ ID NO: 2:

  (i) SEQUENZ CHARAKTERISTIKA:

    (A) LANGE: 24 Basenpaare
    (B) ART: Nukleinsäure

(C) STRANGFORM: Einzel
(D) TOPOLOGIE: linear

(ii) ART DES MOLEKÜLS: cDNS

(xi) SEQUENZBESCHREIBUNG: SEQ ID NO: 2:


GGATCCCCCT  CGGAGGAACC  CGAA                                                      24


(2) INFORMATION ZU SEQ ID NO: 3:

(i) SEQUENZ CHARAKTERISTIKA:

(A) LÄNGE: 22 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 3:


CGTCCCGGGC  GTTTCGGCTC  GC                                                        22


(2) INFORMATION ZU SEQ ID NO: 4:

(i) SEQUENZ CHARAKTERISTIKA:

(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 4:


GGATCCCCCT  CGGAGGAACC  CGGCATGCGG                                                30


(2) INFORMATION ZU SEQ ID NO: 5:

(i) SEQUENZ CHARAKTERISTIKA:

(A) LÄNGE: 28 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 5:


CTGCAGAGCT  CCCCGGCGAG  CGGGAGCC                                                  28

(2) INFORMATION ZU SEQ ID NO: 6:

    (i) SEQUENZ CHARAKTERISTIKA:

        (A) LÄNGE: 24 Basenpaare
        (B) ART: Nukleinsäure
        (C) STRANGFORM: Einzel
        (D) TOPOLOGIE: linear

    (ii) ART DES MOLEKÜLS: cDNS

    (xi) SEQUENZBESCHREIBUNG: SEQ ID NO: 6:

**TTCTGCAGTT CTGGAAAACC GGGC**                                         **24**

(2) INFORMATION ZU SEQ ID NO: 7:

    (i) SEQUENZ CHARAKTERISTIKA:

        (A) LÄNGE: 24 Basenpaare
        (B) ART: Nukleinsäure
        (C) STRANGFORM: Einzel
        (D) TOPOLOGIE: linear

    (ii) ART DES MOLEKÜLS: cDNS

    (xi) SEQUENZBESCHREIBUNG: SEQ ID NO: 7:

**AATCTAGATT ATTTCAGCCC CAGA**                                           **24**

(2) INFORMATION ZU SEQ ID NO: 8:

    (i) SEQUENZ CHARAKTERISTIKA:

        (A) LÄNGE: 24 Basenpaare
        (B) ART: Nukleinsäure
        (C) STRANGFORM: Einzel
        (D) TOPOLOGIE: linear

    (ii) ART DES MOLEKÜLS: cDNS

    (xi) SEQUENZBESCHREIBUNG: SEQ ID NO: 8:

**ATTGCATGCA TATGCAGCTC CTGG**                                           **24**

(2) INFORMATION ZU SEQ ID NO: 9:

    (i) SEQUENZ CHARAKTERISTIKA:

        (A) LÄNGE: 24 Basenpaare
        (B) ART: Nukleinsäure
        (C) STRANGFORM: Einzel
        (D) TOPOLOGIE: linear

(ii) ART DES MOLEKÜLS: cDNS

(xi) SEQUENZBESCHREIBUNG: SEQ ID NO: 9:


**CTGCAGCATT CTGGGCTAGG TGTG**                                                        **24**


(2) INFORMATION ZU SEQ ID NO: 10:

 (i) SEQUENZ CHARAKTERISTIKA:

  (A) LÄNGE: 24 Basenpaare
  (B) ART: Nukleinsäure
  (C) STRANGFORM: Einzel
  (D) TOPOLOGIE: linear

 (ii) ART DES MOLEKÜLS: cDNS

 (xi) SEQUENZBESCHREIBUNG: SEQ ID NO: 10:


 **TTCGGCATGC TTGCTGGGCT CAGT**                                                        **24**


(2) INFORMATION ZU SEQ ID NO: 11:

 (i) SEQUENZ CHARAKTERISTIKA:

  (A) LÄNGE: 24 Basenpaare
  (B) ART: Nukleinsäure
  (C) STRANGFORM: Einzel
  (D) TOPOLOGIE: linear

 (ii) ART DES MOLEKÜLS: cDNS

 (xi) SEQUENZBESCHREIBUNG: SEQ ID NO: 11:


 **AAGCTTTGCG GATTTGCATT TAAT**                                                        **24**


**Beschreibung**

Die Erfindung betrifft ein Verfahren zur Herstellung von rekombinanten Proteinen in Streptomyceten, wobei die Expression in Streptomyces galbus DSM 40480 erfolgt.

Streptomyceten werden in großem Umfang für die industrielle Produktion verschiedener Proteine verwendet. Eine Übersicht über 821 dieser Stämme wird von P. Kämpfer et al. (J. of General Microbiology 137 (1991), 1831-1891) angegeben. Verfahren zur großtechnischen Fermentation von Streptomyceten sind daher weit entwickelt und die Unbedenklichkeit von Streptomyceten für die Herstellung therapeutischer Produkte wurde bereits mehrfach nachgewiesen. Auch für die rekombinante Herstellung von Proteinen sind Streptomyceten geeignet. Für viele verschiedene Streptomyceten-Stämme wurden die hierfür erforderlichen Transformationsverfahren bereits beschrieben (R. Hütter et al. in "Actinomycetes in Biotechnology", eds. Goodfellow, Williams and Mordaski, Academic Press, London, 1988, 89-148; siehe insbesondere Tabelle 10, S. 141-145). Ein Problem bei der Expression heterologer DNA in Streptomyceten stellt jedoch die Spaltung heterologer DNA, z.B. aus E.coli, in den genetisch gut charakterisierten Streptomyceten-Stämmen, wie z.B. Streptomyces coelicolor dar. Von A. Weiß et al. wurde die Expression heterologer Proteine in Streptomyces galbus und Streptomyces lividans beschrieben (6. German VAAM Workshop on the biology of streptomyces, September 30 - October 3, 1990). Streptomyces lividans wird für die Expression heterologer Proteine in Streptomyceten bevorzugt, da er nur eine geringe Spaltungsaktivität gegenüber heterologer DNA aufweist (T. Kieser et al, Meth. Enzymology 204 (1991), 430-458). Die Proteinmenge, die nach Expression in Streptomyces lividans erhalten wird, ist jedoch trotzdem unbefriedigend für eine großtechnische Produktion.

Aufgabe der Erfindung ist es daher, ein Verfahren zur Herstellung von rekombinanten Proteinen in Streptomyceten zur Verfügung zu stellen, bei dem das rekombinante Protein in hoher Ausbeute erhalten wird.

Diese Aufgabe wird gelöst durch ein Verfahren zur Herstellung von rekombinanten Proteinen in Streptomyceten-Zellen, wobei die Zellen mit einem Vektor transformiert werden, der eine für das Protein kodierende DNA-Sequenz enthält, die transformierten Zellen vermehrt werden, wobei das Protein exprimiert wird und das Protein aus den Zellen oder dem Fermentationsüberstand gewonnen wird, wobei dieses Verfahren dadurch gekennzeichnet ist, daß die Expression in Streptomyces galbus DSM 40480 erfolgt.

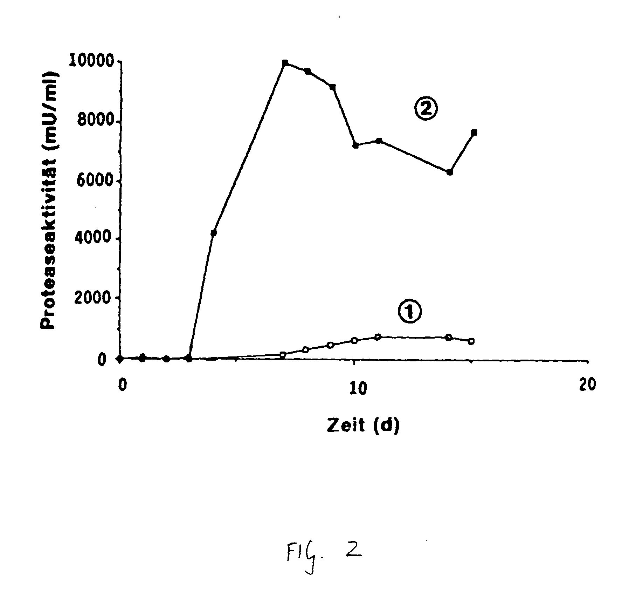

Es hat sich überraschenderweise gezeigt, daß bei Verwendung von Streptomyces galbus DSM 40480 eine wesentlich höhere Ausbeute an rekombinantem Protein erzielt wird als bei Verwendung von Streptomyces lividans. So kann Serinprotease B aus Streptomyces griseus nach Klonierung und Expression in Streptomyces galbus DSM 40480 in 10 - 50-fach höherer Ausbeute erhalten werden, als nach Klonierung und Expression in Streptomyces lividans unter ansonsten identischen Bedingungen (s. Figur 2).

Vorzugsweise werden zur Transformation durch Lysozymbehandlung und Einfrieren bei -70°C kompetente Protoplasten von Streptomyces galbus DSM 40480 hergestellt. Die Transformation dieser kompetenten Protoplasten mit der Vektor DNA kann in Gegenwart von Polyethylenglycol (PEG) durchgeführt werden. Nach der Transformation erfolgt zunächst eine Inkubation für 30 Stunden bei 20-25°C zur Regeneration der Protoplasten, bevor die Selektion transformierter Streptomyceten erfolgt. Zur Expression der heterologen DNA werden die Streptomyceten dann in einem geeigneten komplexen oder synthetischen Medium kultiviert. Die Auswahl der dem Fachmann bekannten Medien richtet sich im wesentlichen nach dem zu produzierenden heterologen Protein und damit nach den anzuwendenden Aufreinigungsschritten. Das rekombinante Protein wird dann aus den Zellen oder dem Fermentationsüberstand nach bekannten Verfahren isoliert.

Vorzugsweise wird für die Herstellung des rekombinanten Proteins ein Expressionsvektor verwendet, bei dem die für das rekombinante Protein kodierende DNA-Sequenz unter der Kontrolle eines starken Promotors steht. Besonders bevorzugt wird der ermE-Promotor (M. Bibb et al., Gene 41 (1986) 357 - 368), des Erythromycin-Resistenzgens (ermE) aus Saccharopolyspora erythreus verwendet. Unter dem ermE-Promotor sind auch Derivate und Allele dieses Promotors zu verstehen, die durch Deletion, Substitution, Insertion, sowie Addition von Nukleotiden erhalten werden und die eine Initiation der Transkription bewirken. Ebenso geeignet sind hybride Promotoren, die Anteile des ermE-Promotors enthalten. Ebenso bevorzugt wird ein Derivat des ermE-Promotors verwendet, das als ermE-up-Promotor bezeichnet wird und dessen Sequenz in SEQ ID NO: 1 angegeben ist.

Weiter ist es bevorzugt, daß die für das rekombinante Protein kodierende DNA-Sequenz unter der Kontrolle eines induzierbaren Promotors steht.

Ein geeigneter induzierbarer Promotor ist z.B. der Gal P1 Promotor aus dem Galactose Operon von Streptomyces lividans (Fornwald et al., Proc. Natl. Accad. Sci. USA 84 (1987), 2130-2134). Vorzugsweise kann auch ein hybrider Promotor z.B. aus dem ermE-up-Promotor und dem Gal P1 Promotor verwendet werden, der eine induzierbare hohe Expression in Streptomyceten ermöglicht.

Streptomyces galbus Stamm DSM 40480, E.coli HB 101 mit pIJ699, (DSM 7031) und E.coli JM83 mit pIJ4070 (DSM 7032) wurden am 03. April 1992 bei der Deutschen Sammlung für Mikroorganismen und Zellkulturen GmbH, Mascheroder Weg 1 b, 3300 Braunschweig, für die Zwecke von Patentverfahren hinterlegt.

Die Erfindung wird durch die folgenden Beispiele in Zusammenhang mit den Abbildungen und den Sequenzprotokollen näher erläutert.

Figur 1 zeigt den Expressionsvektor pCRW69.

Figur 2 zeigt einen Vergleich der Mengen aus Serinprotease B, die nach Expression in Streptomyces lividans 66 TK23 (Kurve 1) bzw. Streptomyces galbus, DSM 40480, (Kurve 2) erhalten werden.

| | |
|---|---|
| SEQ ID NO 1: | zeigt die Sequenz des ermE-up-Promotors. |
| SEQ ID NO 2 und SEQ ID NO 3: | zeigen die zur Klonierung und Mutagenese des sprB-Signalpeptids verwendeten PCR-Primer |
| SEQ ID NO: 4 und SEQ ID NO: 5 | zeigen die zur Klonierung der Serinprotease B aus Streptomyces griseus verwendeten PCR-Primer |
| SEQ ID NO: 6 und SEQ ID NO: 7 | zeigen die zur Klonierung der alkalischen Phosphatase aus E.coli verwendeten PCR-Primer |
| SEQ ID NO: 8 und SEQ ID NO: 9 | zeigen die zur Klonierung der humanen Co-Lipase verwendeten PCR-Primer |
| SEQ ID NO: 10 und SEQ ID NO: 11 | zeigen die zur Klonierung der humanen PankreasAmylase verwendeten PCR-Primer |

**Beispiel 1:**

Konstruktion des Expressionsvektors pCRW69

Der Vektor pIJ699 (T. Kieser et al.,Gene 65 (1988), 83-91) wird mit den Restriktionsendonukleasen KpnI und BglII verdaut und das resultierende 4,27 kb große Fragment mit einer Promotor-Signalpeptid-Multilinker-Kassette ligiert.

Die für das Signalpeptid kodierende Sequenz wird aus dem die Serinprotease B von Streptomyces griseus (DSM 40236) kodierenden Gen sprB gewonnen (Sequenz des sprB-Gens angegeben in: Henderson et al., J. Bacteriol 169 (1987), 3778-3784). Sie umfaßt die für das Signalpeptid kodierende Sequenz, einschließlich der Ribosomenbindestelle und einen Abschnitt nach der Spaltstelle der Signalpeptidase im Genprodukt. Dieser Bereich wird über eine Amplifikation mit partiell nicht homologen Primern (SEQ ID NO: 2 und 3) gewonnen und dabei so verändert, daß er als 150 bp großes Fragment mittels einer BamHI/SmaI Restriktion zugänglich wird.

Dieses BamHI/SmaI Fragment wird in den mit BamHI/HindII geschnittenen Vektor pIJ4070, der den ermE-up Promotor (SEQ ID NO 1) sowie einen Multilinker enthält, ligiert und das so erhaltene Plasmid pCRW68 in E.coli JM83 (Messing et al.,Gene 33 (1985), 103-119) transformiert.

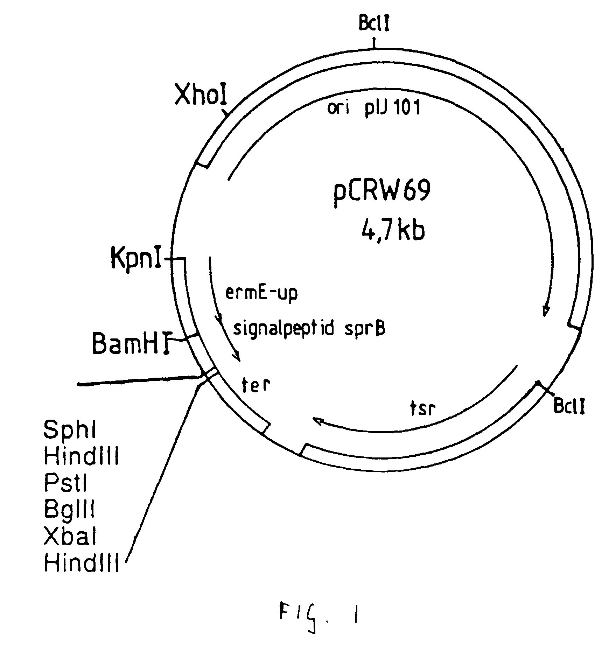

Die so entstandene 420 bp umfassende Promotor-Signalpeptid-Multilinker-Kassette wird als BglII/KpnI-Fragment von pCRW68 in den mit KpnI und BglII geschnittenen Vektor pIJ699 eingefügt, wodurch der Streptomycetenvektor pCRW69 mit erweitertem Polylinker entsteht (Figur 1).

**Beispiel 2:**

Klonierung des Protease B-Gens aus Streptomyces griseus DSM 40236

Der die Serinprotease B von Streptomyces griseus kodierende DNA-Abschnitt, einschließlich Ribosomenbindestelle, Signalpeptid und Propeptid (Sequenz angegeben in: Henderson et al., J. Bacteriol. 169 (1987), 3778-3784) wird aus genomischer DNA von Streptomyces griseus DSM 40236 mittels partiell nicht homologer Oligonukleotidprimer (SEQ ID NO 4 und 5) über PCR amplifiziert und dabei mutagenisiert, so daß dieser Bereich als BamHI/PstI-Fragment zugänglich wird. Dieses 1,0 kb lange BamHI/PstI Fragment wird in den mit BamHI/PstI verdauten Vektor pCRW69 ligiert und der so erhaltene Vektor pCRW70 in Streptomyces galbus transformiert (Beispiel 6). Durch Verdau dieses Vektors pCRW70 mit SphI und einem Enzym, welches im Bereich des Multilinkers schneidet, bietet pCRW70 die Möglichkeit zur Klonierung verschiedener Gene unter der Kontrolle des ermE-up-Promotors.

**Beispiel 3:**

Klonierung des Gens für die Alkalische Phosphatase phoA aus Escherichia coli 294

Das phoA Gen wird als HindIII/XhoI-Fragment aus der DNA von E.coli 294, wie von Chang et al. beschrieben präpariert (Chang et al., Gene 44 (1986), 121-125) und in einen mit HindIII/Sall verdauten pUC18 Vektor (Messing et al., Gene 33 (1985), 103-119) in E.coli JM83 kloniert. Über eine PCR-Mutagenese mit den in SEQ ID NO 6 und 7 gezeigten Primern wird der, die reife alkalische Phosphatase kodierende, Bereich als 1,36 kb langes PstI/XbaI-Fragment amplifiziert und in den mit PstI/XbaI verdauten Vektor pCRW69 (Beispiel 1) ligiert.

**Beispiel 4:**

Klonierung der humanen Co-Lipase-cDNA

Der Genabschnitt für die reife humane Co-Lipase aus Pankreas (Lowe et al., Biochem 29 (1989), 823-828) wird als PCR-Fragment aus einer λgt11-Genbank (Clontech Laboratories, Inc., Palo Alto, CA, USA, Cat. No. HL 1069b) durch Amplifikation erhalten, wobei durch PCR-Mutagenese mit den in SEQ ID NO 8 und 9 gezeigten Primern am 5'-Ende eine SphI-Schnittstelle und am 3'- Ende eine PstI-Schnittstelle eingeführt wird. Das 340 bp lange Fragment wird in den mit SphI/PstI verdauten Vektor pCRW69 (Beispiel 1) ligiert, wobei das Gen im Bereich der Fusionstelle verändert wird.

**Beispiel 5:**

Klonierung der humanen Pankreas-Amylase-cDNA

Die humane pankreatische Amylase cDNA wird wie folgt kloniert: Aus einer käuflichen cDNA-Genbank in λgt11 (Clontech Laboratories, Inc., Palo Alto, CA, USA, Cat. No. HL 1069b) wird die kodierende Sequenz für die reife Amylase (Nakamura et al.,Gene 28 (1984), 263-270 und Nishide et al., Gene 50 (1986), 371-372) mittels PCR amplifiziert und dabei durch Verwendung partiell nicht homologer Primer (SEQ ID NO 10 und 11) so verändert, daß sie als SphI/HindIII-Fragment zugänglich wird. Das 1,53 kb lange SphI/HindIII-Fragment wird in den mit SphI/HindIII verdauten Vektor pCRW69 ligiert.

**Beispiel 6:**

Protoplastierung und Transformation von Streptomyces galbus, DSM 40480

Die Transformation von Streptomyces galbus, DSM 40480, erfolgt in Anlehnung an Chater et al. (Chater et al., Current Topics Microbiol. 46 (1982), 69-95). Zur Protoplastierung wird in einem 250 ml Erlenmeyerkolben eine 25 ml Hauptkultur in TSB (Trypton-Soybroth; Oxoid 30 g/l) oder YEME-Medium (beide mit 10,3 % Sucrose, aber ohne Glycin) mit 2 ml einer stationären Vorkultur von Streptomyces galbus in TSB angeimpft und für 36 Stunden bei 28°C unter intensivem Schütteln inkubiert. Im Anschluß wird die Kultur auf 2 sterile Zentrifugenröhrchen aufgeteilt, 10 Minuten bei 2600 g und 4°C abzentrifugiert und das Mycel 2 x mit einer 10,3 % Sucroselösung gewaschen. Danach werden die Zellen 45 Minuten bei 30°C in 4 ml Lysozym-haltigem (1 mg/ml) P-Puffer inkubiert, die Suspension 3 x mit einer 5 ml Glaspipette auf- und abpipettiert und weitere 15 Minuten inkubiert. Nach Zugabe von 5 ml P-Puffer und Durchmischung mit einer Pipette wie oben, wird durch sterile Watte filtriert. Die so erhaltenen Protoplasten werden abzentrifugiert (7 Minuten, 1500 g) in je 4 ml P-Puffer resuspendiert und in 1 ml Aliquots in 1,5 ml Reaktionsgefäßen bei -70°C langsam auf Eis eingefroren.

Zur Transformation wird ein Aliquot der eingefrorenen Protoplasten bei 37°C schnell aufgetaut, 4 Minuten bei Raumtemperatur und 3000 g abzentrifugiert, das Pellet im verbliebenen Tropfen resuspendiert und mit maximal 5 μl DNA-Lösung versetzt. Nach Zugabe von 100 μl 25 %-igem (w/v) PEG 1000 (Riedel de Haën) in T-Puffer und Mischen durch 3-maliges Auf- und Abziehen durch eine abgeschnittene 200 μl Plastikpipettenspitze verdünnt man sofort mit 850 μl P-Puffer, zentrifugiert wie oben 4 Minuten bei Raumtemperatur und resuspendiert das Pellet in 1 ml P-Puffer.

Zur Regeneration nach der Transformation werden die Protoplasten auf R2YE-Agarplatten (1 Stunde in einer Sterilbank vorgetrocknet) ausplattiert und ca. 30 Stunden bei 20-25°C regeneriert. Nach Überschichten mit Thiostrepton haltigem P-Puffer (300 μg/ml) werden die Kulturen zur Selektion weiter bei 30°C inkubiert.

TSB/10,3 % Sucrose

| | |
|---|---|
| TSB-Fertig Medium (Oxoid) | 30,0 g/l |
| Sucrose | 103,0 g/l |

YEME-Medium/10,3 % Sucrose

| | |
|---|---|
| Hefeextrakt | 3,00 g/l |
| Pepton | 5,00 g/l |
| Malzextrakt | 3,00 g/l |
| Glucose | 10,00 g/l |
| Sucrose | 103,00 g/l |
| $MgCl_2$ x $6H_2O$ | 1,02 g/l |

P-Puffer

| | |
|---|---|
| Sucrose | 103,0 g/l |
| $K_2SO_4$ | 0,20 g/l |
| $MgCl_2$ x$6H_2O$ | 2,02 g/l |
| Spurenelementlösung | 2,0 ml |

destilliertes Wasser ad 800,0 ml
in 80 ml Aliquots autoklavieren und anschließend steril dazupipettieren:

| | |
|---|---|
| $KH_2 PO_4$ (0,5 %) | 1 ml |
| $CaCl_2$ x$H_2O$ (3,68 %) | 10 ml |
| TES-Puffer (5,73 %, pH 7,2) | 10 ml |

TES-Puffer: 5,73 g TES (N-Tris(hydroxymethyl)methyl-2-aminoethanesulfonic acid, SERVA) auf 95 ml destilliertes Wasser einwiegen und pH mit 2 mol/l NaOH auf pH 7,2 einstellen, anschließend auf 100 ml auffüllen und autoklavieren

Spurenelementlösung

| | |
|---|---|
| $ZnCl_2$ | 40,0 mg/l |
| $FeCl_3$ x$6H_2O$ | 200,0 mg/l |
| $CuCl_2$ x$2H_2O$ | 10,0 mg/l |
| $MnCl_2$ | 10,0 mg/l |
| $Na_2B_4O_7$ x 10 $H_2O$ | 10,0 mg/l |
| $(NH_4)_6 Mo_7O_4$x$4H_2O$ | 10,0 mg/l |

T-Puffer

| | |
|---|---|
| Sucrose (10,3 %) | 25,0 ml |
| destilliertes Wasser | 75,0 ml |
| Spurenelementlösung | 0,2 ml |
| $K_2SO_4$ (2,5 %) | 1,0 ml |

in Aliqouts zu 9,3 ml autoklavieren und anschließend steril dazupipettieren: $CaCl_2$ x 6 $H_2O$ (3,68 %) 0,2 ml, TM-Puffer pH 8,0: 0,5 ml

TM-Puffer

1 mol/l Tris mit Maleinsäure (fest) auf pH 8,0 einstellen und autoklavieren.

<u>T-Puffer/25 % PEG</u>

0,5 g PEG 1000 (Riedel de Haën) einwiegen und autoklavieren. Unmittelbar vor dem Gebrauch wieder verflüssigen und 1,5 ml T-Puffer steril zupipettieren.

<u>R2YE-Agar</u>

| | |
|---|---|
| Sucrose | 103,0 g/l |
| $MgCl_2$ | 10,12 g/l |
| Glucose | 10,0 g/l |
| $K_2SO_4$ | 0,25 g/l |
| Casaminoacids | 0,1 g/l |
| Agar-Agar (Bacto-Agar, Fa. Difco) | 25,0 g/l |

auf 800 ml mit destilliertem Wasser auffüllen, autoklavieren und danach steril zugeben:

| | |
|---|---|
| $KH_2 PO_4$ (0,5 %) | 10,0 ml |
| $CaCl_2$ x $2H_2O$ (3,68 %) | 80,0 ml |
| L-Prolin (20,0 %) | 15,0 ml |
| TES-Puffer (5,73 %; pH 7,2) | 100,0 ml |
| Spurenelementlösung | 2,0 ml |
| NaOH (1 mol/l) | 5,0 ml |
| Hefeextrakt (Difco) (10,0 %) | 50,0 ml |

**Beispiel 7:**

Expression rekombinanter Proteine in Streptomyces galbus DSM 40480

Mit den gemäß den Beispielen 2-5 klonierten Gensequenzen wird gemäß Beispiel 6 Streptomyces galbus DSM 40480 transformiert. Die transformierten Bakterien werden unter den folgenden Bedingungen zur Expression der klonierten Gene kultiviert:

a) Expression der Protease B aus Streptomyces griseus

Zur optimalen Produktion der Protease B wird Magermilchminimalmedium (MM-Medium) verwendet. In 1000 ml Erlenmeyerkolben werden 100 ml-Kulturen unter starker Belüftung (Gyrotory Wasserbadschüttler Fa. Brunswick Scientific Company, Modell G 76; Schüttelgeschwindigkeit 8) bei 30°C angezogen. Nach einer Phase von 2-4 Tagen setzt die Bildung der Protease ein, die am Aufklaren des Mediums direkt verfolgt werden kann.

<u>MM-Medium</u>

| | |
|---|---|
| NaCl | 5,00 g |
| $MgSO_4$ | 0,20 g |
| $FeSO_4$ | 0,01 g |
| $(NH_4)_2 SO_4$ | 2,00 g* |
| $CaCl_2$ | 0,36 g* |
| $K_2 HPO_4$ | 0,50 g* |
| Glucose | 10,0 g* |
| Milchpulver | 2,0 g* |

die nicht mit * bezeichneten Salze einwiegen, auf 825 ml mit bidestilliertem Wasser auffüllen, 10 Minuten rühren und dann den pH-Wert auf 7,0 bis 7,5 mit NaOH einstellen. Die weiteren mit * bezeichneten Mediumbestandteile separat in je 25 ml, Glucose in 50 ml, tridestilliertem Wasser lösen, autoklavieren und anschließend steril zur autoklavierten Salzlösung zugeben.

b) Expression der alkalischen Phosphatase aus E.coli

Die Produktion der alkalischen Phosphatase aus E.coli erfolgt in TSB Medium unter Kulturbedingungen, wie sie unter Punkt a für die Protease B beschrieben werden.

c) Expression der humanen Pankreasamylase

Zur Produktion von humaner Pankreasamylase wird der entsprechend transformierte Streptomyces galbus in 100 ml Lechevalier-Medium mit 1 % Stärke unter den in Punkt a) genannten Bedingungen kultiviert.

Lechevalier/1 % Stärke

| Stärke | 10,00 g/l |
|---|---|
| L-Asparagin | 10,00 g/l |
| $ZnSO_4 \times 7\ H_2O$ | 0,02 g/l |
| $MgSO_4 \times 7\ H_2O$ | 0,20 g/l |
| $FeSO_4 \times 7\ H_2O$ | 0,02 g/l |
| $CuSO_4 \times 5\ H_2O$ | 0,02 g/l |
| $K_2HPO_4$ | 0,88 g/l |
| $KH_2PO_4$ | 0,68 g/l |

pH-Wert 7,2

d) Expression der humanen Co-Lipase

Die Expression der humanen Co-Lipase erfolgt wie unter a) beschrieben, jedoch in TSB-Medium/10,3 % Sucrose oder YEME-Medium/10,3 % Sucrose (jeweils ohne Glycin).

**Beispiel 8:**

Vergleich der Expression der Serinprotease B in Streptomyces lividans 66 TK23 und Streptomyces galbus DSM 40480

Das 2,8 kb große BglII-Fragment, des für die Serinprotease B von Streptomyces griseus kodierenden sprB-Gens (Sequenz angegeben in: Henderson et al., J. Bacteriol. 169 (1987), 3778-3784) wird in den mit BamHI geschnittenen pUC18-Vektor kloniert, wodurch der Vektor pAWD7-14 erhalten wird. Aus diesem Vektor wird ein 2,7 kb großes PstI/KpnI Fragment, welches das vollständige sprB-Gen einschließlich dem sprB-Promotor enthält, isoliert und in den ebenfalls mit PstI/KpnI geschnittenen Vektor pIJ702 (Katz et al., J. Gen. Microbiol. 129 (1983), 2703-2714) ligiert. Mit dem so erhaltenen Vektor pAWS7-14-4 wurden Streptomyces galbus DSM 40480, sowie Streptomyces lividans 66 TK23 (Hopwood et al. (1985) in Genetic Manipulation of Streptomyces, A laboratory manual, The John Innes Foundation: Norwich) gemäß dem in Beispiel 6 beschriebenen Verfahren transformiert.

Die Proteaseexpression der transformierten Zellen wurde gemäß dem Verfahren von Delmar et al. (Anal. Biochem. 99 (1979), 316-320 und Geiger (Methods enzymatic assay) Bergmeyer editor (1984), 815-818) bestimmt. Hierzu werden in eine Mikroküvette 70 µl Substratlösung (N-Succinyl-Ala-Ala-Pro-Phe-p-Nitroanilid, 3,12 mg/ml in 50 mmol/l Natriumkaliumphosphatpuffer pH 8), sowie 620 µl 50 mmol/l Natriumkaliumphosphatpuffer pH 8 (50 mmol/l $Na_2HPO_4$, sowie 50 mmol/l $KH_2PO_4$) gegeben, gemischt und 5 Minuten bei 30°C in einer temperierbaren Küvette vorinkubiert. Durch Zugabe von 10 µl Kulturüberstand der zu untersuchenden Bakterienstämme wird die Reaktion gestartet und sofort anschließend die Zunahme der Extinktion bei 405 nm mindestens 5 Minuten lang gemessen. Die Volumenaktivität ergibt sich aus

$$\text{Volumenaktivität} = \frac{\text{E } 405\text{nm/min x Verdünnung x Vges}}{\varepsilon 405\text{nm x d x V probe}}$$

V ges :     Gesamtvolumen (0,7 ml)
V probe :     Probenvolumen (0,01 ml)
d :     Schichtdicke der Küvette (1 cm)
ε405nm :     molarer Extinktionskoeffizient (9,304 $M^{-1}cm^{-1}$)

**Patentansprüche**

1. Verfahren zur Herstellung von rekombinanten Proteinen in Streptomyceten-Zellen, wobei die Zellen mit einem Vektor transformiert werden, der eine für das Protein kodierende DNA-Sequenz enthält, die so erhaltenen transformierten Zellen vermehrt werden, wobei das Protein exprimiert wird und das Protein aus den Zellen oder dem Fermentationsüberstand gewonnen wird, dadurch gekennzeichnet, daß die Expression in Streptomyces galbus DSM 40480 erfolgt.

2. Verfahren nach Anspruch 1, dadurch gekennzeichnet, daß ein Expressionsvektor verwendet wird, bei dem die für das Protein kodierende DNA-Sequenz unter der Kontrolle des ermE-Promotors steht.

3. Verfahren nach Anspruch 1, dadurch gekennzeichnet, daß ein Expressionsvektor verwendet wird, bei dem die für das Protein kodierende DNA-Sequenz unter der Kontrolle des ermE-up-Promotors gemäß SEQ ID NO:1 steht.

4. Verfahren nach Anspruch 1, dadurch gekennzeichnet, daß ein Expressionsvektor verwendet wird, bei dem die für das Protein kodierende DNA-Sequenz unter der Kontrolle eines induzierbaren Promoters steht.

**Claims**

1. Process for the production of recombinant proteins in streptomycetes cells, in which the cells are transformed with a vector that contains a DNA sequence coding for the protein, the transformed cells that are obtained in this way are multiplied during which the protein is expressed and the protein is isolated from the cells or from the fermentation supernatant, wherein the expression is carried out in Streptomyces galbus DSM 40480.

2. Process as claimed in claim 1, wherein an expression vector is used in which the DNA sequence coding for the protein is under the control of the ermE promoter.

3. Process as claimed in claim 1, wherein an expression vector is used in which the DNA sequence coding for the protein is under the control of the ermE-up promoter according to SEQ ID NO: 1.

4. Process as claimed in claim 1, wherein an expression vector is used in which the DNA sequence coding for the protein is under the control of an inducible promoter.


**Revendications**

1. Procédé de préparation de protéines recombinantes dans des cellules de streptomycètes, dans lequel on transforme les cellules à l'aide d'un vecteur qui contient une séquence d'ADN codant pour la protéine, on cultive les cellules transformées ainsi obtenues, tandis que la protéine est exprimée, et on récupère la protéine à partir des cellules ou du surnageant de fermentation, caractérisé en ce que l'expression est réalisée dans Streptomyces galbus DSM 40480.

2. Procédé selon la revendication 1, caractérisé en ce que l'on utilise un vecteur d'expression dans lequel la séquence d'ADN codant pour la protéine est sous le contrôle du promoteur ermE.

3. Procédé selon la revendication 1, caractérisé en ce que l'on utilise un vecteur d'expression dans lequel la séquence d'ADN codant pour la protéine est sous le contrôle du promoteur ermE selon SEQ ID n° :1.

4. Procédé selon la revendication 1, caractérisé en ce que l'on utilise un vecteur d'expression dans lequel la séquence d'ADN codant pour la protéine est sous le contrôle d'un promoteur inductible.

FIG. 1

FIG. 2